Europäisches Patentamt

European Patent Office

Office européen des brevets

(11) Veröffentlichungsnummer: **0 123 079**

**A1**

(12) **EUROPÄISCHE PATENTANMELDUNG**

(21) Anmeldenummer: **84102426.8**

(22) Anmeldetag: **07.03.84**

(51) Int. Cl.³: **A 61 M 5/14**
**F 16 K 5/12**

(30) Priorität: **23.04.83 DE 8312029 U**

(43) Veröffentlichungstag der Anmeldung:
**31.10.84 Patentblatt 84/44**

(84) Benannte Vertragsstaaten:
**AT BE CH DE FR GB IT LI LU NL SE**

(71) Anmelder: **Intermedicat GmbH**
**Gerliswilstrasse 45**
**CH-6020 Emmenbrücke(CH)**

(72) Erfinder: **Lesemann, Egon**
**Rhönstrasse 3**
**D-3501 Körle(DE)**

(74) Vertreter: **von Kreisler, Alek, Dipl.-Chem et al,**
**Patentanwälte Dr.-Ing. Schönwald Dr.-Ing. Eishold; Dr.**
**Fues Dipl.-Chem. von Kreisler; Dipl.Chem. Keller**
**Dipl.-Ing. Selting; Dr. Werner Deichmannhaus am**
**Hauptbahnhof**
**D-5000 Köln 1(DE)**

(54) **Tropfenregelgerät.**

(57) Das Tropfenregelgerät zur Regulierung des Durchlaßquerschnittes einer Flüssigkeitsleitung für intravenöse Flüs-sigkeitsinfusionen und -transfusionen, besteht aus einem Gehäuse (1), das eine mit Schlauchteilen (9, 10) der Flüssig-keitsleitung verbindbare Einlauföffnung (5) und eine Auslauföffnung (6) aufweist und in dessen Ventilraum ein mit einem Verstellorgan ausgestattetes drehbares Küken angeordnet ist. An der Auslauföffnung (4) des Gehäuses (1) ist ein starres, rohrförmiges Griffstück (13) mit offenen Enden befestigt, dessen Hohlraum dem Außendurchmesser des Schlauchteiles (10) an der Auslauföffnung (6) angepaßt ist. Das Verstellorgan besteht dabei aus einer Kreisscheibe (11), deren Drehachse zur Längsachse des Griffstückes (13) quergerichtet ist. Auf diese Weise wird eine sichere und ausreichende "Handlage" zur Einhandbedienung erzielt.

FIG.1

Croydon Printing Company Ltd.

## Tropfenregelgerät

Die Erfindung bezieht sich auf ein Tropfenregelgerät zur Regulierung des Durchlaßquerschnittes einer Flüssigkeitsleitung für intravenöse Flüssigkeitsinfusionen und -transfusionen, bestehend aus einem Gehäuse, das eine mit Schlauchteilen der Flüssigkeitsleitung verbindbare Einlauföffnung und eine Auslauföffnung aufweist und in dessen im wesentlichen zylindrischen Ventilraum ein mit einem Verstellorgan ausgestattetes drehbares Küken angeordnet ist, dessen Drehung die Position mindestens einer Reguliernut mit sich über ihre Länge kontinuierlich änderndem Querschnitt in bezug auf die Flüssigkeitsleitung ändert.

Als Regel- bzw. Abklemmechanismen des Volumenstromes einer Infusions- bzw. Transfusionsflüssigkeit werden häufig Rollenklemmen und Schiebeklemmen benutzt, deren Vorteil darin besteht, daß sie sich mit nur einer Hand bedienen lassen, die jedoch den Nachteil haben, daß sie nicht im Rahmen einer zulässigen Toleranz über einen Zeitraum von einigen Stunden die Tropfenfolge konstant halten können und daß sie schlauchabhängig sind. Zur Behebung dieses Nachteiles wurden die eingangs erwähnten Tropfenregelgeräte geschaffen (DE-GM 77 22 466, DE-OS 27 35 955), die sich durch konstante Tropfenfolge auszeichnen und schlauchunabhängig sind. Solche Tropfenregelgeräte, deren Verstellorgane aus einem Hebel oder einem Rädchen bestehen, lassen sich jedoch nur schwer und unzulänglich mit einer Hand bedienen, so daß eine exakte Einstellung nicht sicher gegeben ist. Damit erfüllen diese Tropfenregelgeräte eine allgemeine klinische Forderung nicht und entsprechen nicht der Handhabungsgewohnheit des Personals.

Der Erfindung liegt die Aufgabe zugrunde, die bekannten Tropfenregelgeräte so zu verbessern, daß eine sichere und ausreichende "Handlage" zur Einhandbetätigung erzielt wird.

Diese Aufgabe wird dadurch gelöst, daß an der Auslauföffnung des Gehäuses ein starres, rohrförmiges Griffstück mit offenen Enden befestigt ist, dessen Hohlraum dem Außendurchmesser des Schlauchteiles an der Auslauföffnung angepaßt ist und daß das Verstellorgan aus einer Kreisscheibe besteht, deren Drehachse zur Längsachse des Griffstückes quergerichtet ist.

Das Griffstück und die mit dem drehbaren Körper verbundene Kreisscheibe machen es möglich, das Tropfenregelgerät so in der Hand zu halten, daß das montierte Griffstück von Mittel-, Ring- und kleinem Finger in der Handinnenfläche gehalten wird und daß mit Daumen und Zeigefinger die Kreisscheibe über den gesamten Regelbereich so gedreht werden kann, bis die gewünschte Tropfgeschwindigkeit erreicht ist. Damit ist die Einhandbedienung des Tropfenregelgerätes gegeben und zusätzlich zu der Forderung der Tropfkonstanz über mehrere Stunden und der Schlauchunabhängigkeit erfüllt es nun auch diese klinische Forderung.

In vorteilhafter Ausgestaltung der Erfindung kann das langgestreckte rohrförmige Griffstück auf der Außenfläche ergonometrisch profiliert sein.

Die Wand des Griffstückes kann umfangsmäßig geschlossen ausgebildet sein. In diesem Falle wird der mit der Auslauföffnung des Gehäuses verbundene Schlauchteil der Flüssigkeitsleitung durch das rohrförmige Griffstück axial hindurchgefädelt. Alternativ kann in der Wand des Griffstückes ein offen endender Längsschlitz ausgebildet sein, durch den der Schlauchteil von der Seite her in den Hohlraum des Griffstückes eingelegt wird. Die Breite des Längsschlitzes ist auf den Außendurchmesser des Schlauchteiles so abgestimmt, daß dieser nicht unbeabsichtigt seitlich aus dem Griffstück austreten kann.

In weiterer vorteilhafter Ausgestaltung der Erfindung ist vorgesehen, daß das Griffstück etwa Handbreitenlänge aufweist und sein Durchmesser in Richtung der Kreisscheibe zunimmt und daß der gesamte Rand der Kreisscheibe mit einer Rändelung versehen ist. Diese Merkmale tragen zur Verbesserung der Handlage bei und erleichtern die Einstellung durch erhöhte Griffigkeit der Kreisscheibe. Die Einstellung ist exakt durchführbar.

An der Kreisscheibe und dem Gehäuse können zusammenwirkende Anschlagelemente vorgesehen sein, die die beiden Endstellungen der Kreisscheibe zur vollen Öffnung

oder Absperrung des Flüssigkeitsdurchlasses fixieren.

Vorteilhafterweise ist vorgesehen, daß das Küken als hohler zylindrischer Rohrkörper gestaltet ist, in dessen Seitenwand zwei gegenüberliegende identische Löcher ausgebildet sind, die in Öffnungsstellung des Kükens mit den Einlauf- und Auslauföffnungen des Gehäuses fluchten und von denen jeweils eine Reguliernut ausgeht, die in der Außenfläche des Kükens ausgeformt ist und deren Tiefe in Umfangsrichtung des Kükens bis zum Übergang in seine Umfangsfläche kontinuierlich abnimmt. Diese Ausbildung ermöglicht eine sehr genaue Einstellung der Tropfrate durch feinfühliges Verstellen der Kreisscheibe um kleine Winkelschritte.

Durch Drehung des Kükens in dem Gehäuse im Gegenuhrzeigersinn werden die Reguliernuten mit der Einlauf- und der Auslauföffnung verbunden und der fluidführende Überschneidungsbereich nimmt bei Drehung des Kükens allmählich zu. Wenn die Reguliernuten bei Drehung des Kükens im Uhrzeigersinn mit ihren an der Umfangsfläche des Kükens mündenden Enden in die Wandzone des Ventilraumes des Gehäuses kommen, ist der Durchfluß gesperrt.

In der Zeichnung sind Ausführungsbeispiele der Erfindung schematisch dargestellt. Es zeigen:

Fig. 1 eine Seitenansicht eines Tropfenregelgerätes mit einer Ausführungsform der Reguliereinrichtung,

Fig. 2 eine Draufsicht des Tropfenregelgerätes nach Fig. 1,

Fig. 3 ein weiteres Ausführungsbeispiel des Tropfenregelgerätes in Draufsicht,

Fig. 4 einen Längsschnitt eines Tropfenregelgerätes mit abgewandelter Reguliereinrichtung in größerem Maßstab,

Fig. 5 einen Querschnitt des Tropfenregelgerätes entlang der Linie V-V und

Fig. 6 einen Querschnitt des Tropfenregelgerätes entlang der Linie VI-VI.

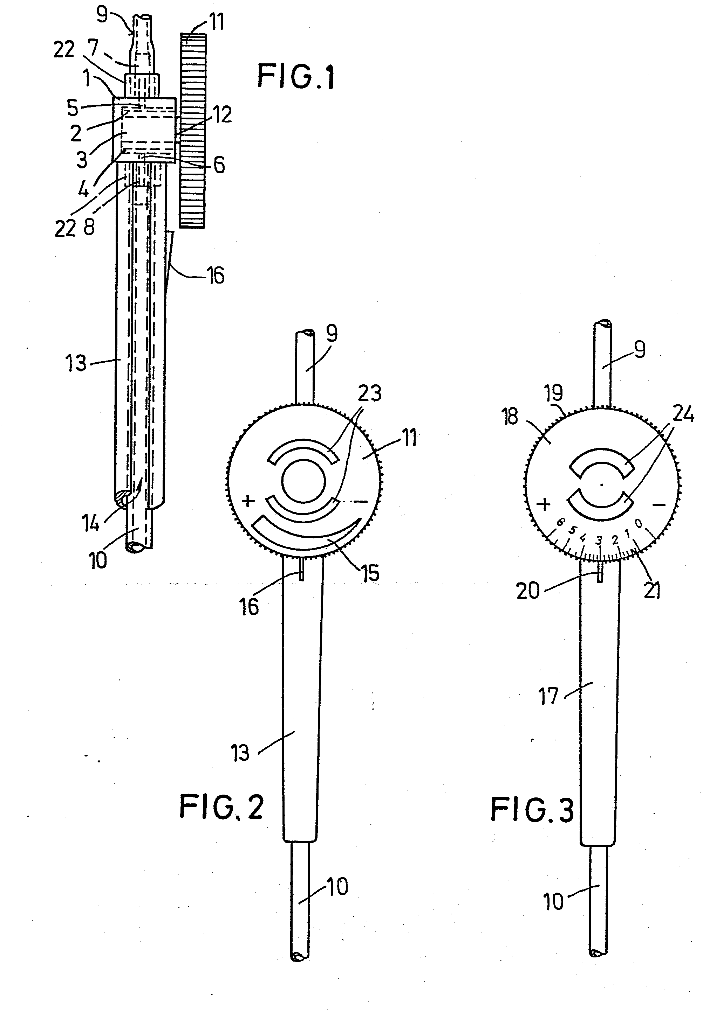

Das Tropfenregelgerät nach Fig. 1 besteht im wesentlichen aus einem einenends geschlossenen Gehäuse 1 mit einem zylindrischen Ventilraum 2, in dem ein passendes zylindrisches Küken 3 angeordnet ist, auf dessen geschlossener Außenfläche eine Reguliernut 4 ausgebildet ist,die sich über den größten Teil seines Umfanges erstreckt und deren Querschnitt sich über ihre Länge radial kontinuierlich ändert. Der Ventilraum 2 steht mit einer Einlauföffnung 5 und einer Auslauföffnung 6 in der Wand des Gehäuses 1 in Verbindung, die sich lotrecht gegenüberliegen und jeweils durch einen Stutzen 7, 8 verlängert sind, auf dem ein flexibler Schlauchteil 9, 10 steckt. Der obere Schlauchteil 9 kommt von einem üblichen Übertragungsgerät, beispielsweise einer Tropfkammer, während der untere Schlauchteil 10 der Flüssigkeitsleitung zum Patienten führt. Durch Drehung des Kükens 3 kann die Reguliernut 4 in bezug auf die Einlauföffnung 5 und die Auslauföffnung 6 so verstellt werden, daß sich eine Änderung des Durchlaßquerschnittes von maximaler Öffnung über allmähliche Drosselung bis zum Verschluß und umgekehrt ergibt.

Als Handhabe bei der Drehung des Kükens 3 dient eine mit seinem einen Ende einstückig ausgebildete Kreisscheibe 11, die auf ihrem gesamten Umfang gerändelt ist und deren Drehachse 12 zur Längsachse der Schlauchteile 9, 10 quergerichtet ist. Die Kreisscheibe 11 liegt daher parallel neben einem starren Griffstück 13, das an der Auslauföffnung 6 auf eine das Schlauchteilende umgebende Hülse 22 klemmend aufgesteckt ist. Das Griffstück 13 hat etwa Handbreitenlänge und besteht aus einem an beiden Enden offenen, geraden Rohr mit kreisförmigem Querschnitt, dessen Innendurchmesser etwa dem Außendurchmesser des Schlauchteiles 10 entspricht. Zum Einlegen des Schlauchteiles 10 in das Griffstück 13 dient ein Längsschlitz 14, der sich über die gesamte Länge des Griffstückes 13 erstreckt. Der Längsschlitz 14 ist in bezug auf das Gehäuse 1 vorzugsweise seitwärts oder nach hinten gerichtet.

Auf der Vorderseite der Kreisscheibe 11 befindet sich ein keilförmiges richtungsweisendes Symbol 15, das mit einer strichförmigen Nase 16 am oberen Ende des Griffstückes 13 als Einstellhilfe bei der Einstellung der gewünschten Tropfenzahl dient (Fig. 2).

Das Griffstück 13 wird mit einer Hand umfaßt und zur Regulierung wird mit Daumen und Zeigefinger der gleichen Hand die Kreisscheibe 11 zur Betätigung des Kükens 3 verdreht. Auf diese Weise ist eine bequeme Einhandbedienung des Tropfenregelgerätes möglich.

Bei dem Tropfenregelgerät gemäß Fig. 3 ist das Griffstück 17 als Rohrhülse mit umfangsmäßig geschlossener Wand ausgebildet. Durch dieses Griffstück 17 wird die Schlauchleitung 10 zum Aufstecken auf den Stutzen 8 des Gehäuses 1 axial hindurchgeschoben. Auf der Kreisscheibe 18, deren Umfang mit einer umlaufenden Rändelung 19 profiliert ist, befindet sich als Einstellhilfe eine Skala 21, die mit einer Nase 20 am oberen Teil des Griffstückes 17 die am Küken 3 eingestellte Tropfrate anzeigt.

Die beiden gebogenen Schlitze 23 bzw. 24 in den Kreisscheiben 11 und 18 der Fig. 2 und 3 erleichtern das Gewicht der Kreisscheiben und tragen zur Materialeinsparung bei.

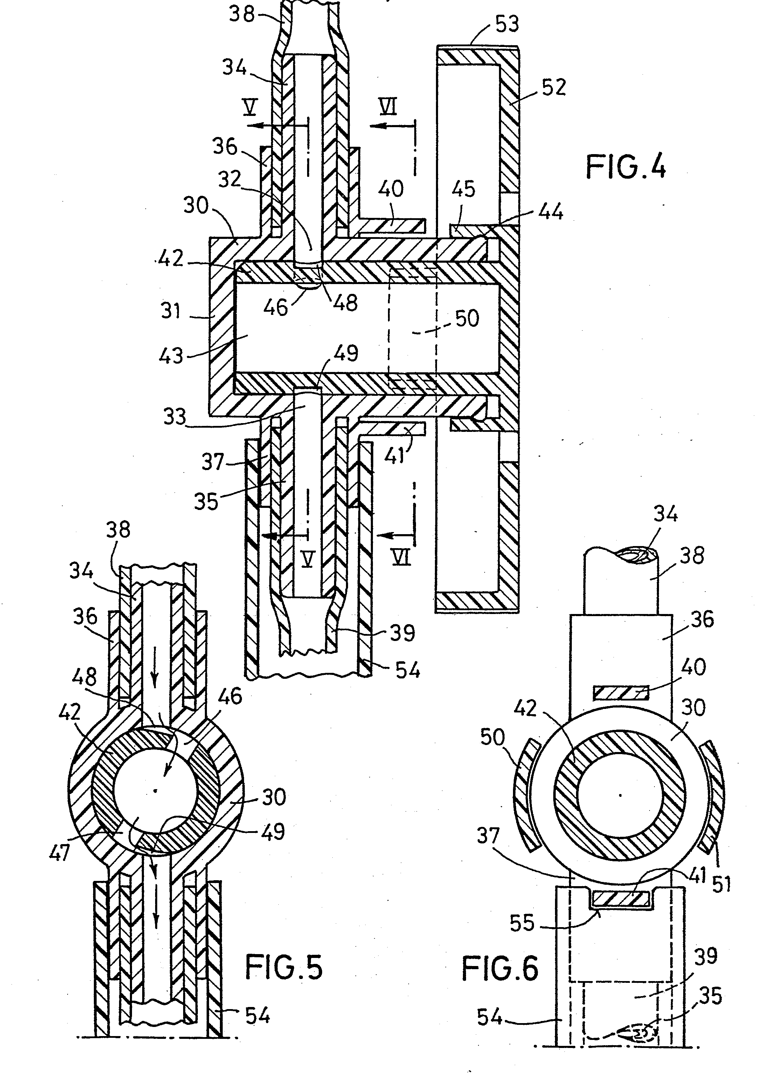

Bei dem Tropfenregelgerät gem. Fig. 4 bis 6 ist eine abgewandelte Reguliereinrichtung vorgesehen. Ein Gehäuse 30 mit zylindrischem Ventilraum ist an einem Ende durch eine Wand 31 verschlossen, während sein anderes Ende offen ist. In seiner Wand sind um 180° zueinander versetzt eine Einlauföffnung 32 und eine Auslauföffnung 33 ausgebildet, die jeweils von einem Stutzen 34, 35 nach außen verlängert sind, die mit umfangsmäßigem Abstand von je einer kürzeren Hülse 36, 37 umgeben sind. Auf jeden Stutzen 34, 35 ist ein flexibler Schlauchteil 38, 39 aufgesteckt, dessen Ende jeweils von dem Zwischenraum zwischen dem Stutzen 34, 35 und der Hülse 36, 37 passend aufgenommen wird. Von jeder Hülse 36, 37 steht eine gerade Zunge 40, 41 ab, die die Außenfläche der Wand des Gehäuses 30 nicht berührt und sich parallel zu dieser in Form einer schmalen ebenen Platte erstreckt.

Durch das offene Ende des Gehäuses 30 ist in seinen zylindrischen Ventilraum ein zylindrisches hohles Küken 42 so weit eingeschoben, daß sein offenes Ende 43 gegen die Innenfläche der Wand 31 des Gehäuses 30 dicht anliegt. In diesem Zustand rastet eine Randwulst 44 auf dem Außenumfang des offenen Endes des Gehäuses 30 in eine angepaßte Ringrille in einer Kappe 45 ein, die auf die Wand des Gehäuses 30 aufgesteckt und mit dem Küken 42 einstückig ausgebildet ist.

In der Seitenwand des Kükens 42 sind zwei identische Löcher 46, 47 (Fig. 5) ausgebildet, deren Form, Durchmesser und Anordnung denjenigen der Einlauf- und Auslauföffnungen 32, 33 des Gehäuses 30 im wesentlichen entsprechen. Wenn das Küken 42 so gedreht ist, daß die Öffnungen 32, 46, 47 und 33 miteinander koaxial fluchten, so ist die größte Durchströmöffnung des Tropfenregelgerätes eingestellt, wobei die Strömung durch den Hohlraum des Kükens 42 hindurchgeht. Zur allmählichen Verringerung bis zum vollständigen Verschluß der Durchströmöffnung dienen zwei Reguliernuten 48, 49, die in der Außenfläche des Kükens 42 ausgebildet sind und sich, ausgehend von einem Loch 46 oder 47, in Umfangsrichtung erstrecken. Die Reguliernut 48, 49 hat etwa rechteckigen Querschnitt und ihre Tiefe nimmt, ausgehend von dem Rand des zugehörigen Loches 46 oder 47 in Umfangsrichtung allmählich ab. Die beiden Reguliernuten 48, 49 sind in bezug aufeinander entgegengesetzt gerichtet und ihre Länge ist so bemessen, daß zur Einstellung von der größten Durchströmöffnung bis zum vollständigen Verschluß eine Drehung des Kükens 42 im Uhrzeigersinn um maximal 90° genügt. Die Verschlußstellung ist erreicht, sobald das in die Umfangsfläche des Kükens 42 übergehende flache Ende jeder Reguliernut 48, 49 in den Bereich der Wandinnenfläche des Gehäuses 30 auf gegenüberliegenden Seiten der Öffnungen 32, 33 gelangt. Zur Begrenzung der 90°-Drehung wirken die Zungen 40, 41 mit gekrümmten Zungen 50, 51 zusammen, die von dem Rand der Kappe 45 axial abstehen und um 180° zueinander versetzt sind.

Das Küken 42, die Kappe 45 und die Zungen 50, 51 sind einstückig mit einer hutförmigen Kreisscheibe 52 ausgebildet, die auf dem Außenumfang ihrer axial gerichteten Wand eine umlaufende Rändelung 53 aufweist. Es ist auch bei diesem Ausführungsbeispiel auf die Hülse 37 an der Auslauföffnung 33 des Gehäuses 30 ein rohrförmiges Griffstück 54
aufgesteckt, das den Schlauchteil 39 über ein etwa
handbreitenlanges Stück versteift und das mit drei Fingern umfaßt werden kann, während Daumen und Zeigefinger
der gleichen Hand die Kreisscheibe 52 verstellen. Im
oberen Rand des Griffstückes 54 ist ein Schlitz 55 ausgebildet, dessen Breite und Tiefe der Zunge 41 angepaßt
ist, so daß sie diese aufnimmt und die Stirnfläche des
Randes des Griffstückes 54 gegen die Außenfläche der
Wand des Gehäuses 30 anliegt. Die in dem Schlitz 55
versenkte Zunge 41, die, wie in Fig. 4 ersichtlich, ein
kurzes Stück über die Außenfläche des Griffstückes 54
vorragt, dient somit nicht nur als Anschlagelement für
die beiden Zungen 50, 51 an der Kreisscheibe 52, sondern sie ist außerdem als Fixierung wirksam, die eine
Drehung des Gehäuses 30 relativ zum Griffstück 54 verhindert, wenn die Kreisscheibe 52 verstellt wird.

ANSPRÜCHE

1. Tropfenregelgerät zur Regulierung des Durchlaßquerschnittes einer Flüssigkeitsleitung für intravenöse Flüssigkeitsinfusionen und -transfusionen, bestehend aus einem Gehäuse, das eine mit Schlauchteilen der Flüssigkeitsleitung verbindbare Einlauföffnung und eine Auslauföffnung aufweist und in dessen im wesentlichen zylindrischen Ventilraum ein mit einem Verstellorgan ausgestattetes drehbares Küken angeordnet ist, dessen Drehung die Position mindestens einer Reguliernut mit sich über ihre Länge kontinuierlich änderndem Querschnitt in bezug auf die Flüssigkeitsleitung ändert, d a d u r c h   g e k e n n z e i c h n e t,   daß an der Auslauföffnung (4;33) des Gehäuses (1;30) ein starres, rohrförmiges Griffstück (13;17;54) mit offenen Enden befestigt ist, dessen Hohlraum dem Außendurchmesser des Schlauchteiles (10;39) an der Auslauföffnung (6;33) angepaßt ist und daß das Verstellorgan aus einer Kreisscheibe (11;52) besteht, deren Drehachse zur Längsachse des Griffstückes (13;17;54) quergerichtet ist.

2. Tropfenregelgerät nach Anspruch 1, d a d u r c h   g e k e n n z e i c h n e t, daß das Griffstück auf der Außenfläche ergonometrisch profiliert ist.

3. Tropfenregelgerät nach Anspruch 1 oder 2, d a d u r c h   g e k e n n z e i c h n e t, daß die Wand des Griffstückes (17;54) umfangsmäßig geschlossen ausgebildet ist.

4. Tropfenregelgerät nach Anspruch 1 oder 2,
d a d u r c h   g e k e n n z e i c h n e t, daß
in der Wand des Griffstückes (13) ein offen endender
Längsschlitz (14) ausgebildet ist.

5. Tropfenregelgerät nach einem der Ansprüche 1 bis 4,
d a d u r c h   g e k e n n z e i c h n e t, daß
das Griffstück (13;17;54) etwa Handbreitenlänge aufweist und sein Durchmesser in Richtung der Kreisscheibe
(11;18;52) zunimmt und daß der gesamte Rand der Kreisscheibe (11;18;52) mit einer Rändelung versehen ist.

6. Tropfenregelgerät nach einem der Ansprüche 1 bis 5,
d a d u r c h   g e k e n n z e i c h n e t, daß
an der Kreisscheibe (52) und dem Gehäuse (30) zusammenwirkende Anschlagelemente (40,41;50,51) vorgesehen
sind.

7. Tropfenregelgerät nach einem der Ansprüche 1 bis 6,
d a d u r c h   g e k e n n z e i c h n e t,   daß
in dem Rand des Griffstückes (54) ein Schlitz (55) ausgebildet ist, der eines der Anschlagelemente (41) am
Gehäuse (30) aufnimmt.

8. Tropfenregelgerät nach Anspruch 1,
d a d u r c h   g e k e n n z e i c h n e t,   daß
das Küken (42) als hohler zylindrischer Rohrkörper gestaltet ist, in dessen Seitenwand zwei gegenüberliegende identische Löcher (46,47) ausgebildet sind, die in
Öffnungsstellung des Kükens (42) mit den Einlauf- und
Auslauföffnungen (32,33) des Gehäuses (30) fluchten und
von denen jeweils eine Reguliernut (48,49) ausgeht, die
in der Außenfläche des Kükens (42) ausgeformt ist und
deren Tiefe in Umfangsrichtung des Kükens (42) bis zum
Übergang in seine Umfangsfläche kontinuierlich abnimmt.

9. Tropfenregelgerät nach Anspruch 8,
d a d u r c h     g e k e n n z e i c h n e t,     daß
die Umfangslänge von dem flachen Ende der Reguliernut
(48,49) bis zum gegenüberliegenden Rand des zugehörigen
Loches (46,47) etwa 45° beträgt.

FIG.1

FIG.2

FIG.3

0123079

**FIG.4**

**FIG.5**

**FIG.6**

Europäisches
Patentamt

EUROPÄISCHER RECHERCHENBERICHT

0123079
Nummer der Anmeldung

EP 84 10 2426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | WO-A-8 200 327 (WHITMAN MEDICAL) * Seite 5, Zeilen 7-21; Seite 7, Zeilen 25-31; Seite 8, Zeilen 18-22; Abbildungen * | 1-3,5, 6 | A 61 M 5/14 F 16 K 5/12 |
| Y | | 4,8,9 | |
| | --- | | |
| Y | GB-A-2 097 514 (BESPAK INDUSTRIES) * Abbildung 3 * | 4 | |
| | --- | | |
| Y | CH-A- 340 681 (J.S. SCHATT) * Seite 2, Zeilen 18-31; Abbildungen * | 8,9 | |
| | --- | | RECHERCHIERTE SACHGEBIETE (Int. Cl. ³) |
| A,D | DE-A-2 735 955 (MATTHIAS FAESEN) * Abbildungen * | 1,3,5, 8 | |
| | --- | | A 61 M F 16 K |
| A | US-A-3 982 534 (T.P. BUCKMAN) * Spalte 5, Zeilen 44-66; Abbildungen 2,3 * | 1,3 | |
| | --- | | |
| A | US-A-4 361 147 (J.L. ASLANIAN et al.) * Abbildungen 1,4,16,22-24; Spalte 10, Zeilen 25-45 * | 1,4-6 | |
| | --- -/- | | |

Der vorliegende Recherchenbericht wurde für alle Patentansprüche erstellt.

| Recherchenort DEN HAAG | Abschlußdatum der Recherche 31-07-1984 | Prüfer WOLF C.H.S. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-C-3 139 030 (B. BRAUN MELSUNGEN) <br> * Zusammenfassung * <br> --- | 1,2,4-6 | |
| A | GB-A- 818 646 (H. KOPPERS) <br> * Abbildungen 2,3 * <br> ----- | 6,8 | |

RECHERCHIERTE SACHGEBIETE (Int. Cl. ³)

Der vorliegende Recherchenbericht wurde für alle Patentansprüche erstellt.

| Recherchenort <br> DEN HAAG | Abschlußdatum der Recherche <br> 31-07-1984 | Prüfer <br> WOLF C.H.S. |
|---|---|---|